# EUROPEAN PATENT APPLICATION

(11) **EP 4 450 060 A1**
(43) Date of publication of application: **23.10.2024**
(21) Application number: 22907954.6
(22) Date of filing: 15.12.2022
(51) Int. Cl.: A61K 9/00, A61K 31/40, A61P 1/04

(54) **DOSAGE REGIMEN FOR FEXUPRAZAN INJECTION COMPOSITION**

(30) Priority: 15.12.2021 KR 20210179757
(71) Applicant: Daewoong Pharmaceutical Co., Ltd., Hwaseong-si, Gyenoggi-do 18623 (KR)
(72) Inventor: LEE, Areum, Seoul 04734 (KR); HAN, Kihun, Seoul 06170 (KR); LIM, Kwon Jo, Seoul 06170 (KR); BAEK, Song, Gunpo-si, Gyeonggi-do 15836 (KR)
(74) Representative: Germain Maureau
(86) International application number: PCT/KR2022/020425
(87) International publication number: WO 2023/113487

(57) **Abstract**

The present invention provides an appropriate dosage regimen for each indication of a fexuprazan injection composition and thereby enable obtaining an optimal preventive or therapeutic effect therefrom.

## Description

### [TECHNICAL FIELD]

The present invention relates to an appropriate dosage regimen for fexuprazan injection composition.

### [BACKGROUND]

The annual rate of hospital admissions due to acute upper gastrointestinal bleeding in the United States is 160 per 100,000 populations, exceeding 400,000 cases per year. Almost the majority (80-90%) of upper gastrointestinal bleeding cases are non-variceal bleeding, of which the most common lesion is described as a gastroduodenal peptic ulcer. The most common cause of upper gastrointestinal bleeding is peptic ulcer, which accounts for about 50%, and peptic ulcers caused by Helicobacter are the most common cause of upper digestive tract bleeding. Peptic ulcers occur when attack factors such as stomach acid and pepsin are superior to defense factors such as mucus, and is defined as a defect in the gastrointestinal mucosa caused by the attack factors. Common causes of peptic ulcers include Helicobacter infection, use of non-steroidal anti-inflammatory drugs, and physical stress, and in cases of some latrogenic ulcers, ulcers may occur due to endoscopic mucosal resection or endoscopic submucosal dissection.

Therapeutic methods for these ulcerative bleeding include endoscopic therapy, pharmacotherapy, or surgical therapy, wherein the pharmacotherapy is mainly performed via rapid and potent inhibition of gastric acid secretion using injections. Stomach acid damages the blood coagulation process, promotes the breakdown of platelet aggregation, and acts advantageous on fibrinolysis. Therefore, suppressing gastric acid secretion, increasing the intragastric acidity, and maintaining elevated pH even at night promotes stabilization of the blood coagulation process and decreases the tendency to rebleed. However, since high-dose injections of PPI (proton-pump inhibitor) irreversibly act on the intragastric proton pump, there were problems that the proton pump of newly generated parietal cells could not be sustainably suppressed, and it was difficult to obtain meaningful treatment results due to early drug resistance.

P-CAB (potassium-competitive acid blocker) overcomes the drawbacks of PPI, which is an existing gastric acid secretion inhibitor, and reversibly binds to the proton pump, suppresses gastric acid secretion, has high stability against gastric acid, and acts directly on the proton pump rather than as a pro-drug. Thereby, this is opening the market for a new gastric acid secretion inhibitor, based on its advantages such as rapid onset of action, powerful effect, long half-life, and the ability to administer the drug without affecting meals. Substances such as Vonoprazan and Tegoprazan have been commercialized, and are mainly used to treat gastric acid-related diseases, especially gastroesophageal reflux disease (GERD).

Nevertheless, since injections for P-CAB preparations has not been developed, there have been difficulties in administering it to patients who need faster effects than oral drugs or who cannot take the oral drugs.

"1-(5-(2,4-difluorophenyl)-1-((3-fluorophenyl)sulfonyl)-4-methoxy-1H-pyrrol-3-yl)-N-methylmethanamine", named fexuprazan, is a compound described in Korean Patent No. 10-1613245, and has excellent antiulcer activity (i.e., proton pump inhibitory activity, etc.) and Helicobacter pylori (H. pylori) eradication activity and GPCR inhibition activity, whereby it is known to be useful for prevention and treatment of gastrointestinal track ulcers, gastritis, reflux esophagitis, or gastrointestinal damage caused by Helicobacter pylori.

It is known that fexuprazan has therapeutic effects on diseases associated with gastric acid secretion, specifically gastroesophageal reflux disease (GERD), and more specifically, erosive esophagitis (EE), when orally administered at a dose of 40 mg once a day.

Therefore, the present inventors have developed a fexuprazan injection composition and conducted intensive research on the composition, and found an appropriate dosage regimen for each indication of the fexuprazan injection composition, thereby completing the present invention.

### [DETAILED DESCRIPTION OF THE INVENTION]

### [Technical Problem]

It is an object of the present invention to provide an appropriate dosage regimen for each indication of a fexuprazan injection composition.

### [Technical Solution]

In order to achieve the above object, according to the present invention, there is provided an injection composition for preventing or treating gastrointestinal disease in which a compound represented by the following Chemical Formula 1 or a pharmaceutically acceptable salt thereof is administered at a dose of 0.4 to 0.6 times the dose at which the therapeutic effect for oral administration was confirmed:

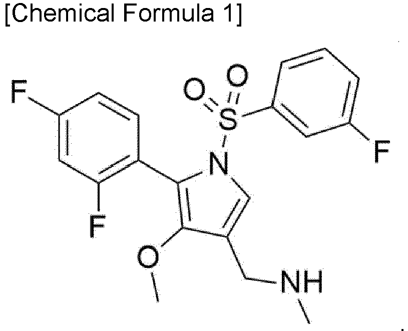

The chemical name of the compound represented by Chemical Formula 1 is 1-(5-(2,4-difluorophenyl)-1-((3-fluorophenyl)sulfonyl)-4-methoxy-1H-pyrrol-3-yl)-N-methylmethanamine, which is a compound described in Korean Patent No. 10-1613245.

The above compound is an active ingredient that exhibits pharmacological effects of the injection composition according to the present invention, and has excellent anti-ulcer activity (i.e., proton pump inhibitory activity, etc.), Helicobacter pylori (H. pylori) eradication activity and GPCR inhibition action, and thus is useful in the prevention and treatment of gastrointestinal track ulcer, gastritis, reflux esophagitis, or gastrointestinal damage caused by Helicobacter pylori.

Further, the above compound as well as a pharmaceutically acceptable salt of the compound can also be used in the present invention. As salts, salts commonly used in the art, such as acid addition salts formed by pharmaceutically acceptable free acids can be used without limitation. The term "pharmaceutically acceptable salt" as used herein refers to any organic or inorganic addition salt of the compound represented by Chemical Formula 1, whose concentration is relatively non-toxic and harmless to a patient and activates effectively and whose side effects do not degrade the beneficial efficacy of the above compound.

Pharmaceutically acceptable salts can be obtained by conventional methods using inorganic or organic acids. For example, the pharmaceutically acceptable salt can be prepared by dissolving the compound represented by Chemical Formula 1 in a water-miscible organic solvent, e.g., acetone, methanol, ethanol or acetonitrile, followed by adding an organic acid or an inorganic acid, and filtering and drying the precipitated crystals. Alternatively, it may be prepared by subjecting a solvent or an excessive amount of acid from the acid-added reaction mixture to reduced pressure and then drying the residue, or by adding a different organic solvent and then filtering the precipitated salt. At this time, the preferred salts may include salts derived from hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid or toluenesulfonic acid, and the like.

On the other hand, the pharmaceutical composition of the present invention can be formulated according to a standard pharmaceutical practice. These formulations may contain additives such as pharmaceutically acceptable carrier, adjuvant or diluent in addition to the active ingredient. In particular, a suitable carrier for preparing an injection composition can be used, and examples thereof include physiological saline, polyethylene glycol, ethanol, vegetable oil, and isopropyl myristate and the like. Diluents include, for example, lactose, dextrose, sucrose, mannitol, sorbitol, cellulose and/or glycine and the like, but are not limited thereto. Further, it can be dissolved in oils, propylene glycol or other solvents commonly used in the preparation of injection compositions.

As used herein, the term "prevention" refers to any act to delay or inhibit occurrence, spread or recurrence of the above-mentioned diseases by administration of the injection composition of the present invention, and "treatment" refers to any act to improve or change the symptoms of the above diseases for the better by administration of the injection composition of the present invention.

Preferably, the injection composition containing the compound represented by Chemical Formula 1 or a pharmaceutically acceptable salt thereof is administered in an amount of 10 mg to 30 mg once a day for the prevention or treatment of gastrointestinal disease. At this time, the mass of 10 mg to 30 mg refers to the amount of the compound represented by Chemical Formula 1 to be administered, and when a pharmaceutically acceptable salt of the compound represented by Chemical Formula 1 is used, it means the amount excluding the salt component. Additionally, the administration is performed preferably through intravenous route.

The gastrointestinal disease may be a disease associated with gastric acid secretion. The disease associated with gastric acid secretion collectively refer to digestive system diseases associated with gastric acid, such as gastric ulcer, duodenal ulcer, erosive reflux esophagitis, and non-erosive gastroesophageal reflux disease. By suppressing or eliminating gastric acid secretion, which is the cause of the disease, it is possible to completely cure diseases or alleviate symptoms. Gastroesphageal reflux disease (GERD) is a condition in which unpleasant symptoms and complications are caused by a regurgitation of stomach contents into the esophagus, and is classified into erosive esophagitis (EE) and non-erosive reflux disease (NERD) on the basis of the presence or absence of morphological changes in the esophagus, such as ulcers or erosions. The erosive esophagitis is defined as a case where there is endoscopically and visually identifiable damage to the distal esophageal mucosa, and the non-erosive is defined as a case where there are typical regurgitation symptoms but no damage to the esophageal mucosa on endoscopy.

In the present invention, it was confirmed that when the condition of pH 4 or more is maintained for 40% or more of the day, it exhibits a therapeutic effect on diseases associated with gastric acid secretion, specifically gastroesophageal reflux disease, and more specifically, erosive esophagitis. When the compound represented by Chemical Formula 1 or a pharmaceutically acceptable salt thereof is orally administered in an amount of 40 mg once a day, it exhibits a therapeutic effect on diseases associated with gastric acid secretion, specifically gastroesophageal reflux disease, and more specifically erosive esophagitis. In the present invention, it was confirmed that when the same compound is administered at 10 mg to 30 mg once a day through intravenous route, it exhibits the same effect as oral administration.

Preferably, the injection composition containing the compound represented by Chemical Formula 1 or a pharmaceutically acceptable salt thereof is administered at 20 mg once a day for the prevention or treatment of gastrointestinal disease.

In addition, according to the present invention, there is provided an injection composition for treating bleeding in which the compound represented by Chemical Formula 1 or a pharmaceutically acceptable salt thereof is administered in an amount of 70 mg to 90 mg once a day, or 30 mg to 50 mg twice a day. At this time, each mass of 70 mg to 90 mg and 30 mg to 50 mg means the amount of the compound represented by Chemical Formula 1 to be administered, and when a pharmaceutically acceptable salt of the compound represented by Chemical Formula 1 is used, it means the amount excluding the salt component.

Gastric acid makes the blood clotting process difficult, and dissolves aggregated platelets and fibrin. Therefore, in order to treat bleeding, the intragastric pH must be maintained high by regulating gastric acid secretion. As the intragastric pH is higher and is maintained longer, the therapeutic effect on bleeding is better. However, it is generally known that when the intragastric acidity pH is 6 or more, the blood coagulation process is stabilized and rebleeding is reduced. When the condition of pH 6 or more is maintained for 50% or more of the day, it exhibits a therapeutic effect. In the present invention, it was confirmed that when the injection composition containing the compound represented by Chemical Formula 1 or a pharmaceutically acceptable salt thereof is administered in an amount of 70 mg to 90 mg once a day, or 30 mg to 50 mg twice a day, such conditions are satisfied. Furthermore, the bleeding may be caused by peptic ulcers, or may be caused by latrogenic ulcers after endoscopic surgery.

Preferably, the injection composition containing the compound represented by Chemical Formula 1 or a pharmaceutically acceptable salt thereof is administered in an amount of 80 mg once a day, or 40 mg twice a day for the treatment of bleeding.

In addition, according to the present invention, there is provided an injection composition for preventing bleeding in which the compound represented by Chemical Formula 1 or a pharmaceutically acceptable salt thereof is administered in an amount of 30 mg to 50 mg, and then maintained and administered at 1 mg/h to 10 mg/h for 24 hours or more. Wherein, the mass of 30 mg to 50 mg and 1 mg/h to 10 mg/h means the amount of the compound represented by Chemical Formula 1 to be administered, and when a pharmaceutically acceptable salt of the compound represented by Chemical Formula 1 is used, it means the amount excluding the salt component. In addition, as a method of maintaining and administering, there is generally mentioned a method of diluting the fexuprazan injection composition in physiological saline (bag), connecting it to an infusion set, and then adjusting the administration speed and administration time.

In order to prevent bleeding, the intragastric pH must be maintained above a certain level, and generally, if the condition of pH 6 or more is maintained for 50% or more of the day, it exhibits a preventive effect. In the present invention, it was confirmed that when an injection composition containing the compound represented by Chemical Formula 1 or a pharmaceutically acceptable salt thereof is maintained and administered in an amount of 30 mg to 50 mg, and then maintained and administered at 1 mg/h to 10 mg/h for 24 hours or more, such conditions is satisfied.

Preferably, the injection composition containing the compound represented by Chemical Formula 1 or a pharmaceutically acceptable salt thereof is administered in an amount of 40 mg and then maintained and administered at 1~10 mg/h for 48 hours or more for the prevention of bleeding.

### [Advantageous Effects]

As mentioned above, the present invention provides an appropriate dosage regimen for each indication of a fexuprazan injection composition and thereby enable obtaining an optimal preventive or therapeutic effect therefrom.

### [BRIEF DESCRIPTION OF THE DRAWINGS]

FIG. 1 schematically illustrates the clinical trial method of Experimental Example 4.
FIG. 2 shows the plasma concentrations during single intravenous administration of 10 mg, 20 mg, 40 mg, and 80 mg of fexuprazan, respectively. FIG. 2(a) is shown on a linear scale, and FIG. 2(b) is shown on a semi-log scale.
FIG. 3 is a diagram showing the relationship between dose and plasma concentration during intravenous administration of fexuprazan injection. For the log value of the fexuprazan dose in each dose group, FIG. 3(a) is a power model graph showing the Cₘₐₓ value in plasma, FIG. 3(b) is a power model graph showing the AUCₗₐₛₜ value in plasma, and FIG. 3(c) is a power model graph showing the log value of the AUC_{inf} value in plasma.
FIG. 4 shows the results of dose correction for Cₘₐₓ, AUCₗₐₛₜ, and AUC_{inf} values following single administration of 10 mg, 20 mg, 40 mg and 80 mg of fexuprazan injection, respectively. For the fexuprazan doses, FIG. 4(a) is a power model graph showing Cₘₐₓ, FIG. 4(b) is a power model graph showing AUCiast, and FIG. 4(c) shows a dose-normalized comparison graph in which the AUC_{inf} parameter values are corrected for each administered dose.
FIG. 5 shows the change in acidity in the gastrointestinal tract over time during single administration of 10 mg, 20 mg, 40 mg, and 80 mg of fexuprazan injection, respectively.
FIG. 6 schematically illustrates the clinical trial method of Experimental Example 5.
FIG. 7 shows the change in acidity in the gastrointestinal tract over time when 40 mg of fexuprazan was administered orally and intravenously, respectively.
FIG. 8 shows an outline of the test of dose groups 1 and 3 in Experimental Example 6 of the present invention.
FIG. 9 shows an outline of the test of dose group 2 in Experimental Example 6 of the present invention.
FIG. 10 shows the acidity (pH) test results in the gastrointestinal tract for dose group 1 in Experimental Example 6 of the present invention.
FIG. 11 shows the acidity (pH) test results in the gastrointestinal tract for dose group 2 in Experimental Example 6 of the present invention.
FIG. 12 shows the acidity (pH) test results in the gastrointestinal tract for dose group 3 in Experimental Example 6 of the present invention.
FIG. 13 shows the time for which the pH in the gastrointestinal tract maintains above 4 in Experimental Example 6 of the present invention.
FIG. 14 shows the time for which the pH in the gastrointestinal tract maintains above 6 in Experimental Example 6 of the present invention.
FIG. 16 shows the test outline of Experimental Example 7 of the present invention.

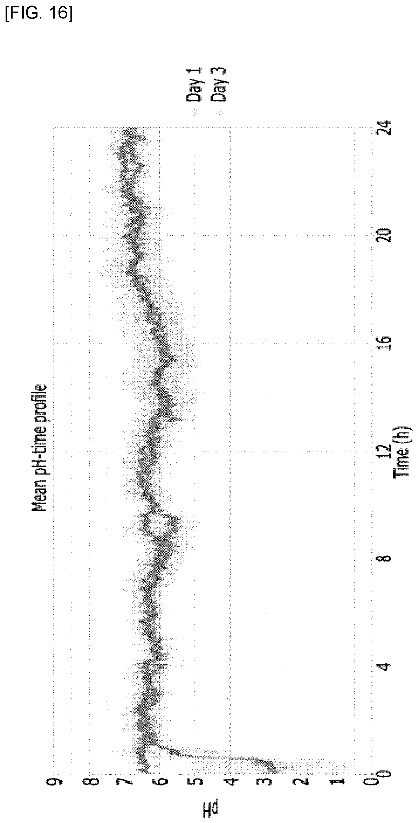

FIG. 16 shows the change in acidity in the gastrointestinal tract over time in Experimental Example 7 of the present invention.

### [DETAILED DESCRIPTION OF THE EMBODIMENTS]

Below, the present invention will be described in more detail to assist in the understanding of the invention. However, the following examples are provided for illustrative purposes only, and should not be construed as limiting the scope of the present invention to these examples.

### Preparation Example

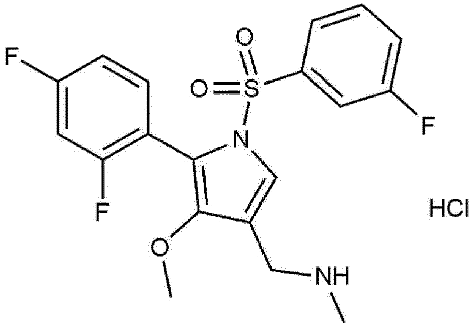

The compound represented by Chemical Formula 1 of the present invention was prepared in the same manner as in Example 8 of Korean Patent Application No. 10-2016-0013588.
Molecular weight: 446.87
¹H-NMR (500 MHz, MeOD): 7.69(s, 1H), 7.58-7.53(m, 1H), 7.45(t, 1H), 7.30(d, 1H), 7.20-7.15(m, 2H), 7.02-6.94(m, 2H), 4.07(d, 2H), 3.46(s, 3H), 2.71(s, 3H)

### Experimental Example 1: Confirmation of bleeding/ulcer therapeutic effect through fexuprazan injection

In order to evaluate the efficacy of fexuprazan against bleeding gastric ulcers, an indomethacin-induced bleeding gastric ulcer model using male rats was used.

Specifically, the previously prepared 1-(5-(2,4-difluorophenyl)-1-((3-fluorophenyl)sulfonyl)-4-methoxy-1H-pyrrol-3-yl)-N-methylmethanamine (hereinafter referred to as 'fexuprazan' hydrochloride was dissolved in a solution containing 30 wt.% of polyethylene glycol (Polyethylene Glycol 400) polymer in physiological saline. The concentration was adjusted so that 0, 0.1, 0.5, 1, 2 mg/kg based on fexuprazan weight could be administered to each test animal in a liquid volume of 1 mL/kg, and then each test animal was administered through the tail vein as a single dose. In addition, 1 mg/kg of esomeprazole magnesium trihydrate, which is a positive control, based on esomeprazole weight was administered as a single dose through the tail vein in test animals. After 30 minutes, 80 mg/kg of indomethacin was orally administered as a single dose to each test animal to induce gastric bleeding and ulceration. An autopsy was performed 5 hours after administration of indomethacin, and the stomach was removed. Then, the ulcer index (%) was measured using an image program (Leica Application Suite V4), and gastric hemorrhage and erosion state were evaluated through histopathological examination. The results are shown in Table 1 below.

**[Table 1]**

| Group | Ulcer index (%) | Gastric hemorrhage state | Gastric erosion state |
|---|---|---|---|
| Negative control | 0.0 ± 0.0 | 0.0 ± 0.0 | 0.0 ± 0.0 |
| Vehicle control 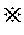 | 10.6 ± 2.3** | 2.0 ± 1.3** | 2.5 ± 1.0** |
| Esomeprazole 1 mg/kg 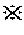 | 6.1 ± 2.6^{##} | 1.3 ± 1.6 | 2.0 ± 1.8 |
| Fexuprazan 0.1 mg/kg 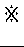 | 9.2 ± 2.3 | 1.5 ± 1.6 | 2.9 ± 1.2 |
| Fexuprazan 0.5 mg/kg 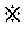 | 4.0 ± 1.8^{##} | 0.5 ± 0.8^{##} | 1.3 ± 1.0^{#} |
| Fexuprazan 1 mg/kg 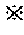 | 4.2 ± 2.5^{##} | 0.3 ± 0.5^{##} | 0.8 ± 0.7^{##} |
| Fexuprazan 2 mg/kg 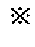 | 4.2 ± 1.5^{##} | 0.3 ± 0.5^{##} | 0.3 ± 0.5^{##} |
| Data are expressed in mean ± SD; statistical analysis was done using SPSS statistic 25.0 and p values were set at level; *Compared with G1: **p<0.01; #Compared with G2: #p<0.05, ##p<0.01; 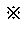 : Indomethacin-induced group; Negative control: 5% Sodium bicarbonate + 30% PEG400 in saline; Vehicle control: 30% PEG400 in saline. | | | |

As shown in Table 1, it was confirmed that a fexuprazan-administered group had a statistically significant decrease in ulcer index and gastric hemorrhage and erosion state compared to an excipient-administered group, thereby exhibiting gastric protection and bleeding prevention effects, and the minimum effective dose was 0.5 mg/kg.

### Experimental Example 2: Fexuprazan injection pharmacokinetic experiment

Methylcellulose was dissolved in physiological saline to prepare a solution containing methylcellulose at a concentration of 0.5 wt%, and then the previously prepared fexuprazan hydrochloride was added thereto to prepare a composition with Fexuprazan at a concentration of 1 mg/mL. The composition prepared above was orally administered to male rats at a dose of 2 mg/kg in a liquid volume of 2 mL/kg depending on the body weight of the individual, and then blood was collected using an orbital blood collection method at 0.25, 0.5, 1, 2, 4, 6, 8, and 24 hours, and centrifuged at 10,000 rpm for 5 minutes. Plasma was collected, analyzed by HPLC-MS/MS, and pharmacokinetic parameters were calculated.

Then, methylcellulose was dissolved in physiological saline to prepare a solution containing methylcellulose at a concentration of 0.5 wt,%, and then the previously prepared fexuprazan hydrochloride was added thereto to prepare a composition with fexuprazan at a concentration of 1 mg/mL. The composition was administered through the tail vein in male rats at a dose of 1 mg/kg in a liquid volume of 1 mL/kg depending on the body weight of the individual. Blood was collected by the same method as in oral administration at 0.083, 0.25, 0.5, 1, 2, 4, 6, 8, and 24 hours, and then analyzed. The analysis results are shown in Table 2 below.

**[Table 2]**

| Parameter | Dose | |
|---|---|---|
| | IV 1 mg/kg | PO 2 mg/kg |
| T_{1/2} (h) | 2.06 ± 0.25 | 1.74 ± 0.16 |
| CL (mL/min/kg) | 114 ± 29 | - |
| V_{d} (mL/kg) | 20200 ± 5200 | - |
| Tₘₐₓ (h) | - | 0.833 ± 0.289 |
| Cₘₐₓ (ng/mL) | - | 23.8 ± 10.7 |
| AUCₗₐₛₜ (ng·h/mL) | 143 ± 38 | 70.9 ± 22.4 |
| BA (%) | 24.7 | |

### Experimental Example 3: Fexuprazan injection toxicity test results

The previously prepared fexuprazan hydrochloride was dissolved in a solution containing 30 wt.% of polyethylene glycol (Polyethylene Glycol 400) polymer in physiological saline, and the concentration was calculated based on fexuprazan weight so that it could be administered at a liquid volume of 5 mL/kg for rats and 2 mL/kg for beagle dogs to prepare each composition. Toxicity was evaluated by intravenously administering at doses of 0, 2.5, 5, and 10 mg/kg/day to rats and beagle dogs, respectively, once a day for 4 weeks. As a result of the test, no toxic symptoms due to the test substance were observed in all administration groups, and thus a high dose of 10 mg/kg/day was set as the harmless dose.

**[Table 3]**

| Test items (Animal type, administration period, administration route) | Dosage (mg/kg) | Result (mg/kg) |
|---|---|---|
| Rat, 4 weeks, intravenous | 0, 2.5, 5, 10 | harmless dose 10 |
| Beagle dog, 4 weeks, intravenous | 0, 2.5, 5, 10 | harmless dose 10 |

### Experimental Example 4: Confirmation of single dose safety/PK/PD of fexuprazan injection

In order to confirm the safety/PK/PD of the compound of the present invention during intravenous administration, the clinical trial was designed with random assignment, double-blind, placebo-controlled, single dose, and stepwise doseescalation in healthy adults (Korean/Chinese) between the ages of 19 and 50.

A stabilizer (200 mg), a freeze-drying auxiliary agent (200 mg), and the previously prepared fexuprazan hydrochloride (40 mg based on fexuprazan weight) were dissolved in water for injection (4 mL), and an appropriate amount of acid was added thereto to adjust the pH to 4.0. The prepared solution was put into a vial, dried by a freeze-drying method, and prepared as a freeze-drying agent.

The freeze-dried composition prepared as above was used, but a total of 28 subjects of 4, 8, 8, and 8 subjects each were enrolled for four dose groups (10 mg, 20 mg, 40 mg, and 80 mg) based on fexuprazan weight, and each group was randomly assigned to test or control groups in the order that they passed the screening criteria at a ratio of 3:1. All test subjects completed safety, pharmacokinetic, and pharmacodynamic evaluations according to the planned clinical trial schedule.

**[Table 4]**

| | Test group | Control group | Number of subjects (test group : control group) | Gende r | Nationality | Random |
|---|---|---|---|---|---|---|
| Dose group 1 | Fexuprazan 10 mg | physiological saline 100 ml | 4(3:1) | male | Korean | Test group |
| | | | | male | Korean | Test group |
| | | | | male | Korean | Test group |
| | | | | male | Korean | Control group |
| Dose group 2 | Fexuprazan 20 mg | physiological saline 100 ml | 8(6:2) | male | Korean | Test group |
| | | | | male | Korean | Test group |
| | | | | female | Korean | Test group |
| | | | | male | Korean | Control group |
| | | | | male | Korean | Test group |
| | | | | female | Chinese | Test group |
| | | | | male | Chinese | Control group |
| | | | | male | Chinese | Test group |
| Dose group 3 | Fexuprazan 40 mg | physiological saline 100 ml | 8(6:2) | male | Korean | Test group |
| | | | | male | Chinese | Control group |
| | | | | male | Korean | Test group |
| | | | | male | Korean | Test group |
| | | | | male | Chinese | Control group |
| | | | | male | Chinese | Test group |
| | | | | male | Korean | Test group |
| | | | | male | Korean | Test group |
| Dose group 4 | Fexuprazan 80 mg | physiological saline 100 ml | 8(6:2) | male | Korean | Test group |
| | | | | male | Korean | Test group |
| | | | | male | Korean | Test group |
| | | | | male | Korean | Control group |
| | | | | male | Chinese | Test group |
| | | | | male | Chinese | Test group |
| | | | | male | Korean | Test group |
| | | | | male | Korean | Control group |

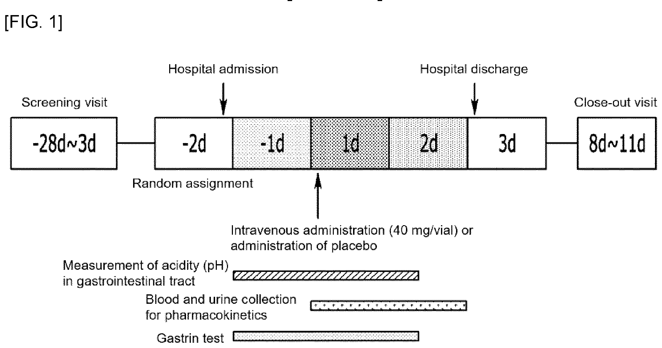

As shown in FIG. 1, a screening visit was performed 3 to 28 days before the start of the clinical trial, and hospital admission was performed 2 days before medication. The medication was performed in the morning, and the patient was discharged from the hospital on the third day of medication. Subsequently, a close-out visit was performed between the 8th and 11th day of medication.

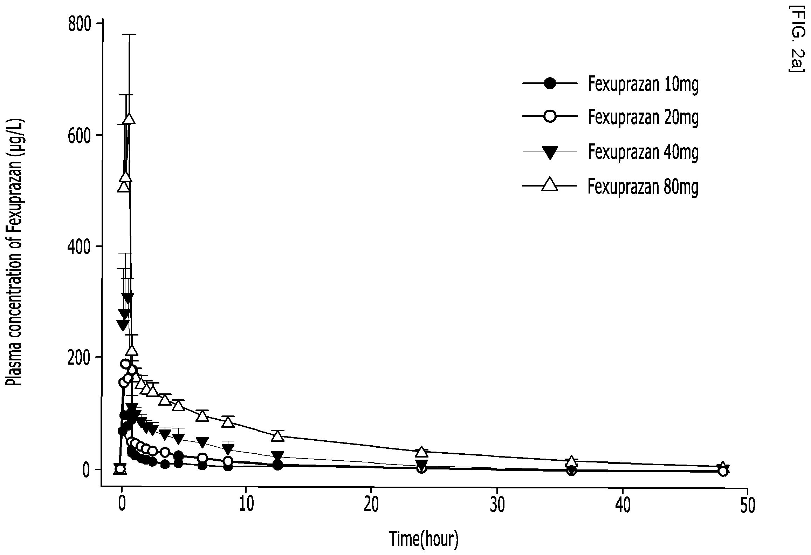

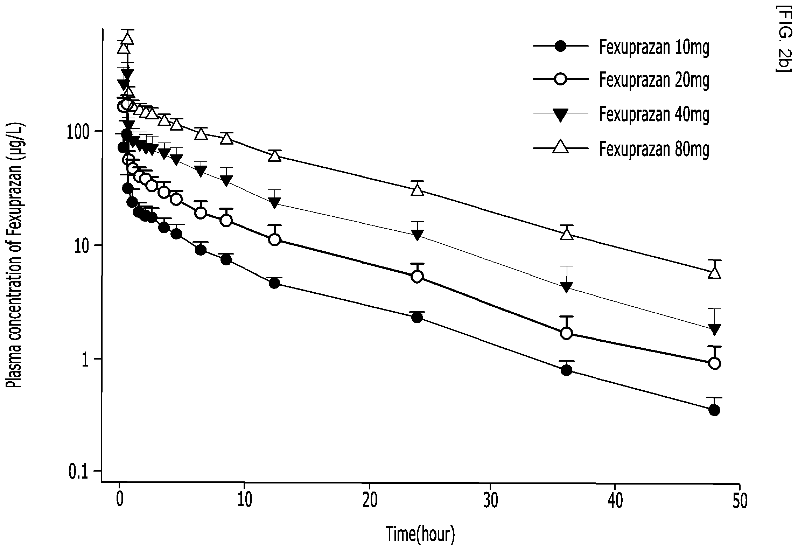

Testing of the acidity (pH) in the gastrointestinal tract and gastric secretion promoting hormone gastrin were performed from 24 hours before medication to 24 hours after medication. Blood collection for pharmacokinetics and urine collection for pharmacokinetics were performed for 48 hours from the time of medication. The measured pharmacokinetic parameters are shown in Tables 5 to 8 and FIGS. 2 to 5, and no serious abnormalities or side effects were observed.

**[Table 5]**

| Parameter | Dose Group | | | |
|---|---|---|---|---|
| | 10 mg (N=3) | 20 mg (N=6) | 40 mg (N=6) | 80 mg (N=6) |
| Cₘₐₓ (ug/L) | 93.7 ± 13.9 | 185 ± 32.3 | 321 ± 75.7 | 633 ± 165 |
| AUCₗₐₛₜ (ug*h/L) | 235 ± 32.3 | 502 ± 111 | 1070 ± 225 | 2290 ± 271 |
| AUC_{inf} (ug*h/L) | 240 ± 31.3 | 514 ± 113 | 1100 ± 238 | 2380 ± 301 |
| t_{1/2} (h) | 9.04 ± 0.85 | 8.61 ± 1.89 | 8.84 ± 0.96 | 10.2 ± 1.84 |
| C_{L} (L/h) | 42.1 ± 5.50 | 40.6 ± 9.30 | 37.8 ± 7.23 | 34.0 ± 4.42 |
| V_{d}/F (L) | 553 ± 120 | 498 ± 134 | 476 ± 74.3 | 496 ± 88.5 |

**[Table 6]**

| PD Parameter | Dose Group | | | | |
|---|---|---|---|---|---|
| | 10 mg (N=3) | 20 mg (N=6) | 40 mg (N=6) | 80 mg (N=6) | Placebo (N=7) |
| Fraction time pH ≥ 4 (%) | 20.4 ± 2.6 | 46.7 ± 19.0 | 71.0 ± 10.3 | 92.9 ± 4.8 | 4.50 ± 4.4 |
| Fraction time pH ≥ 6 (%) | 2.9 ± 2.0 | 16.7 ± 14.6 | 38.3 ± 9.0 | 59.7 ± 15.7 | 1.2 ± 1.3 |
| Mean pH | 2.7 ± 0.1 | 3.9 ± 0.8 | 4.9 ± 0.3 | 5.9 ± 0.4 | 2.0 ± 0.2 |
| Median pH | 2.1 ± 0.2 | 3.8 ± 1.3 | 5.4 ± 0.6 | 6.2 ± 0.3 | 1.7 ± 0.1 |

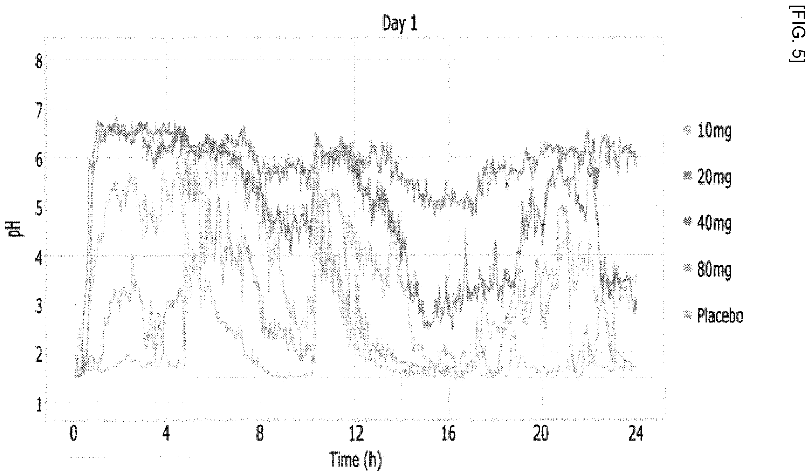

As shown in Table 6 and FIG. 5, as a result of checking the acidity (pH) in the gastrointestinal tract, it was confirmed that after intravenous administration of 80 mg, the time during which the pH maintained above 6 for 24 hours was 59.65%. It was confirmed that during intravenous administration of 40 mg, the time during which the pH maintained above 6 for 12 hours was 50% or more. Therefore, it can be confirmed that it is effective in treating bleeding at doses of 80 mg or more when administered once a day and at doses of 40 mg or more when administered twice a day.

Likewise, as a result of checking the acidity (pH) in the gastrointestinal tract as shown in Table 6 and FIG. 5, it was confirmed that the time during which the pH maintained above 4 for 24 hours after intravenous administration of 20 mg was 46.7%. Accordingly, it can be confirmed that administration of 20 mg once a day exhibits a therapeutic effect on diseases associated with gastric acid secretion.

**[Table 7]**

| Parameter | Slope of regression line (95% Confidence Interval)* |
|---|---|
| Cₘₐₓ | 0.89080 (0.74774 - 1.03386) |
| AUCₗₐₛₜ | 1.09869 (0.98844 - 1.20895) |
| AUC_{inf} | 11.10612 (0.99403 - 1.21820) |
| * In(Y) = a+ b * In(dose), where Y is the PK parameter of interest | |

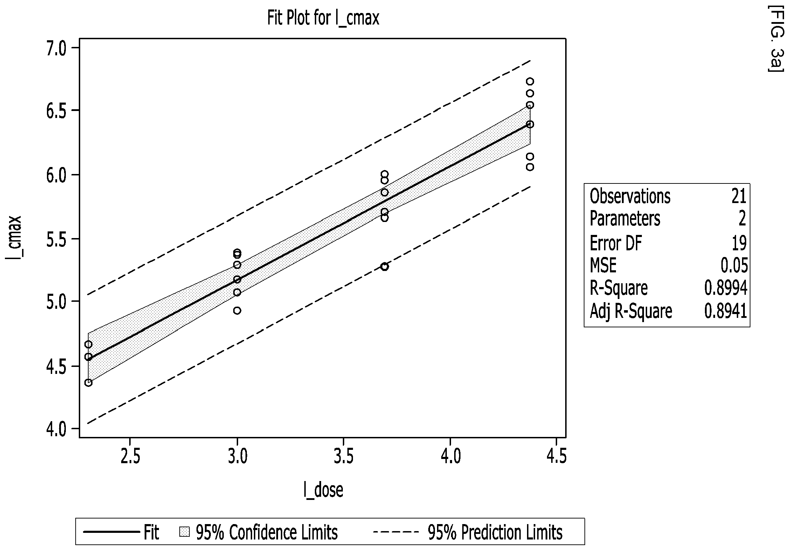

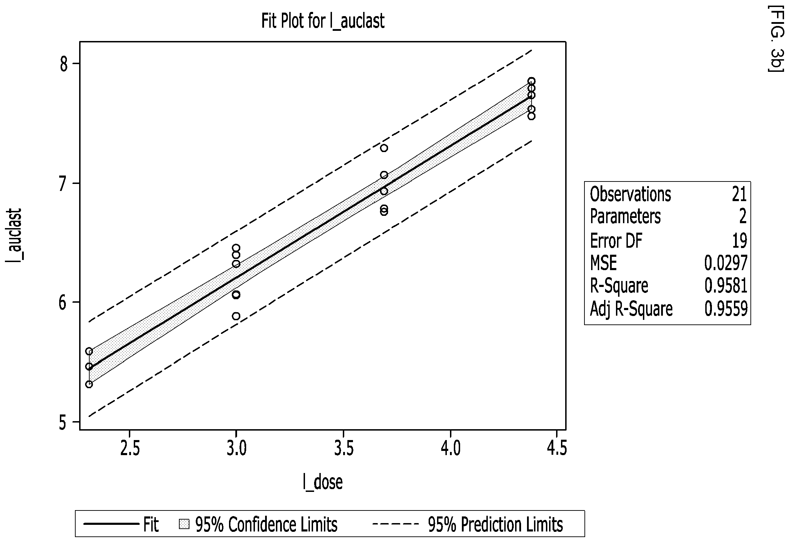

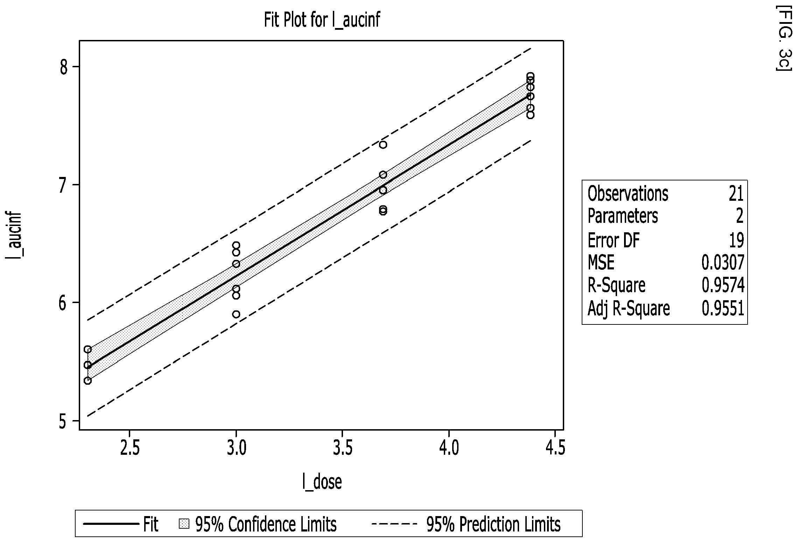

After a single intravenous administration of four dose groups for fexuprazan, the dose proportionality of fexuprazan to the Cₘₐₓ, AUCₗₐₛₜ, and AUC_{inf} parameters was evaluated between doses of 10 mg and 80 mg. The log-transformed parameters and 95% confidence interval regression lines of each dose after a single administration are shown in Table 7 and FIG. 3, respectively. When evaluating single intravenous doses from 10 mg to 80 mg using the power model, the slopes (95% confidence interval) of the regression lines for Cₘₐₓ, AUCₗₐₛₜ, and AUC_{inf} were 0.89 (0.7477 ~ 1.0339), 1.10 (0.9884 ~ 1.2090), and 1.11 (0.9940 ~ 1.2182), respectively, showing an increase in proportion to the dose. That is, it can be confirmed that the pK parameter of intravenous administration is proportional to the dose.

**[Table 8]**

| Parameter | Cₘₐₓ/Dose | AUCₗₐₛₜ/Dose | AUCₗₐₛₜ/Dose |
|---|---|---|---|
| Kruskal-Wallis test P-value | 0.4045 | 0.3430 | 0.2961 |

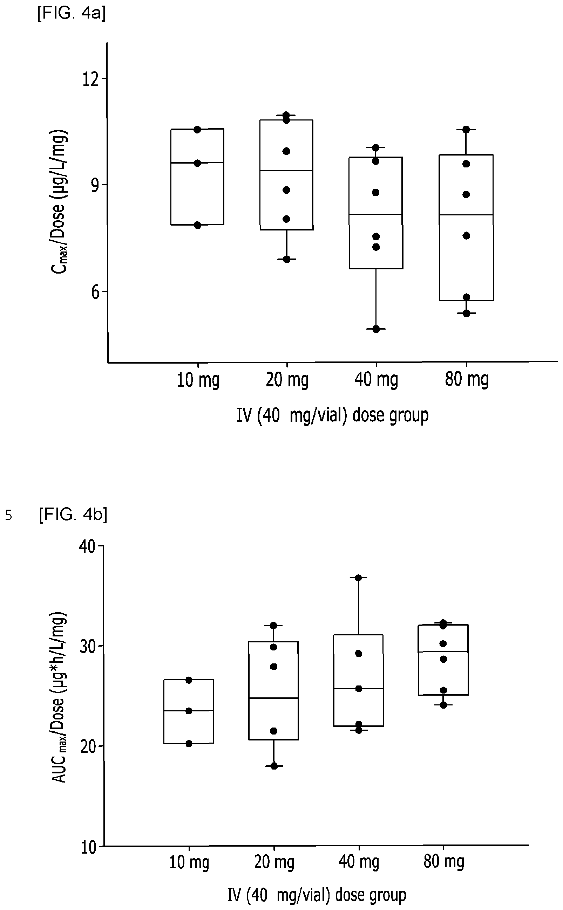

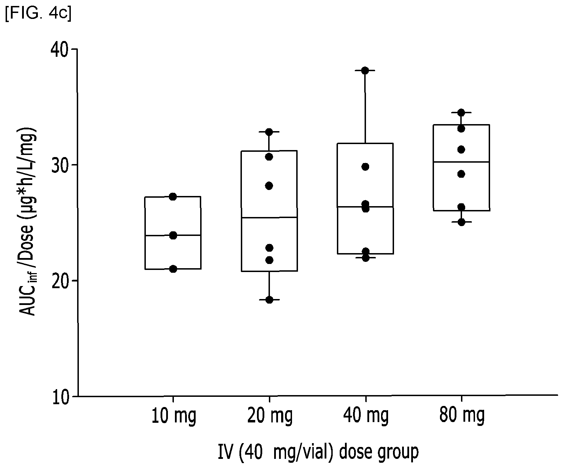

Table 8 and FIG. 4 show the results of the Kruskal Wallis test for dose-normalized Cₘₐₓ (Cₘₐₓ/Dose), AUCₗₐₛₜ (AUCₗₐₛₜ/Dose), and AUC_{inf} (AUC_{inf}/Dose) between dose groups. No statistical significance was observed in any case (p=0.4045, 0.3430, 0.2961). That is, it can be seen that there is no significant difference between dose groups in the proportional relationship, and the appropriate dose for intravenous administration can be calculated indirectly through the appropriate dose for oral administration.

Accordingly, a clinical test was conducted to confirm the bioavailability of the oral drug required to calculate the intravenous dose compared to the oral dose.

### Experimental Example 5: Confirmation of bioavailability (BA) of fexuprazan injection

The dosage regimen for injection preparations usually compares pharmacokinetic parameters such as bioavailability and peak blood concentration for oral administration versus intravenous administration, and pharmacological parameters associated therewith, which was applied to the dosage regimen for oral administration, thereby being able to infer an appropriate regimen for injection preparations.

Therefore, in order to compare the safety/PK/PD of the compound of the present invention during single intravenous administration and during single oral administration and derive the bioavailability, the clinical trial was designed as a randomized, open-label, single-dose, two-period, two-way crossover trial in healthy adults. The selection/exclusion criteria were the same as in Experimental Example 4. A total of 8 subjects were enrolled, and four subjects each were randomly assigned to sequence group 1 or sequence group 2 in the order in which they passed the screening criteria. All test subjects completed safety, pharmacokinetic, and pharmacodynamic evaluations according to the planned clinical trial schedule.

**[Table 9]**

| Experimental group | Gender | Nationality |
|---|---|---|
| Sequence group 1 (Intravenous administration -> oral administration) | Male | Korean |
| | Female | Korean |
| | Male | Chinese |
| | Female | Chinese |
| Sequence group 2 (Oral administration -> intravenous administration) | Male | Korean |
| | Male | Korean |
| | Female | Korean |
| | Male | Korean |

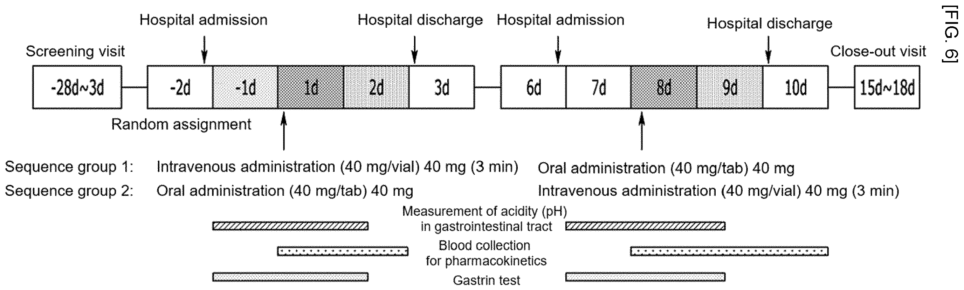

A screening visit was performed 3 to 28 days before the start of the clinical trial, and hospital admission was performed 2 days before medication. The first phase medication was performed according to the order group randomly assigned on the morning of the day. A stabilizer (200 mg), a freeze-drying auxiliary agent (200 mg), and the previously prepared fexuprazan hydrochloride (40 mg based fexuprazan weight) were dissolved in water for injection (4 mL), an appropriate amount of acid was added thereto. A composition whose pH was adjusted to 4.0 was administered intravenously, or an excipient (100 mg), a disintegrant (5 mg), and the previously prepared fexuprazan hydrochloride (40 mg based on fexuprazan weight) were mixed and compressed to prepare a tablet, which were administered orally. The hospital discharge was performed on the third day of medication. The second phase medication was performed on the morning of the 8th after hospital admission on the 6th day after the first phase medication. Hospital discharge was performed on the 10th day of medication. Subsequently, a close-out visit was performed between 15 and 18 days after medication (FIG. 6).

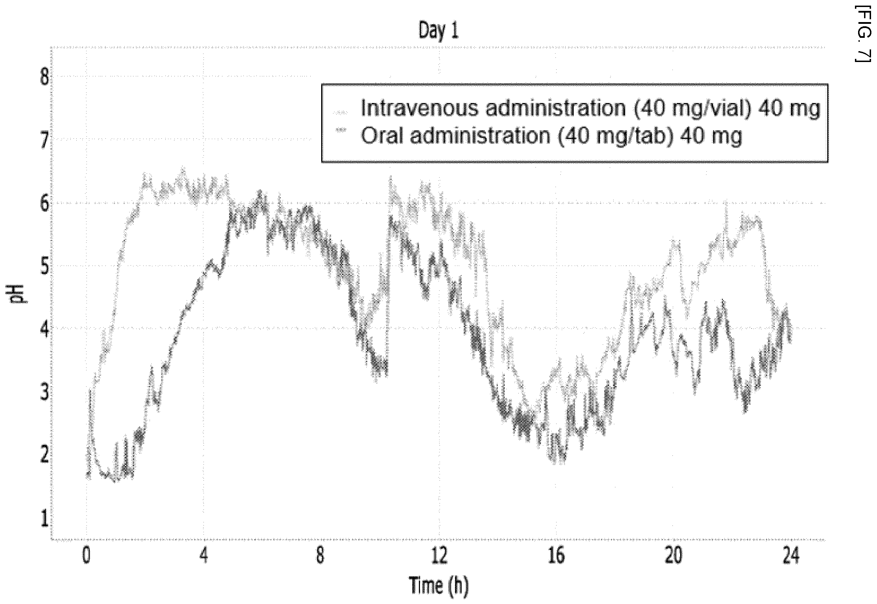

Testing of the acidity (pH) in the gastrointestinal tract and gastric secretion promoting hormone gastrin were performed from 24 hours before medication to 24 hours after medication. Blood collection for pharmacokinetics and urine collection for pharmacokinetics were performed for 48 hours from the time of medication. The measured pharmacokinetic parameters are shown in Tables 10 and 11 and FIG. 7, and no serious abnormalities or side effects were observed.

**[Table 10]**

| Item | Oral administration (40 mg) | Intravenous administration (40 mg) |
|---|---|---|
| n | 8 | 8 |
| Cₘₐₓ (µg/L) | 49.9 ± 23.4 | 277 ± 291 |
| AUCₗₐₛₜ (µg·h/L) | 536 ± 278 | 1110 ± 235 |
| AUC_{inf} (µg·h/L) | 556 ± 299 | 1150 ± 279 |
| Tₘₐₓ (h) | 2.09 ± 0.706 | 0.25 ± 0 |
| t_{1/2} (h) | 9.38 ± 1.6 | 9.53 ± 2.42 |
| F (AUCₗₐₛₜ) | 0.46 ± 0.142 | 1± 0 |
| F (AUC_{inf}) | 0.458 ± 0.137 | 1± 0 |

**[Table 11]**

| Item | Oral administration (40 mg) | Intravenous administration (40 mg) |
|---|---|---|
| n | 8 | 8 |
| Fraction time pH≥4 (%) | 47.5 ± 14.2 | 71.6 ± 13.5 |
| Fraction time pH≥6 (%) | 20.3 ± 12.8 | 38.7 ± 10.9 |
| Mean pH | 3.9 ± 0.7 | 4.9 ± 0.5 |
| Median pH | 3.9 ± 1.2 | 5.5 ± 0.7 |

As can be seen in Table 10, it was found that when 40 mg of fexuprazan was administered orally and intravenously, the Cₘₐₓ values were 49.9 µg/L and 277 µg/L, respectively, and the Tₘₐₓ values were 2.09 h and 0.25 h, respectively. Through this, the injection drug reached a higher peak blood concentration (Cₘₐₓ) in a faster time than the oral drug. The AUCₗₐₛₜ values were 536 µg·h/L and 1110 µg·h/L, respectively. Through this, it was confirmed that the bioavailability during oral administration was about 0.46.

PD parameters are shown in Table 11 above. As a result of checking the acidity (pH) in the gastrointestinal tract, it was confirmed that the percentage of time during which the pH maintained above 4 was 47.5% and 71.6% during oral and intravenous administrations, respectively. The percentage of time during which the pH maintained above 4 was 20.3% and 38.7% during oral and intravenous administrations, respectively. In addition, the mean pH values for oral and intravenous administration were 3.9 and 4.9, respectively.

Taking into consideration the results of Tables 10, 11, and FIG. 7, it can be seen that the medicinal efficacy (PD) of fexuprazan is related to the blood concentration(PK) of fexuprazan, and intravenous administration has a faster Tₘₐₓ and higher Cₘₐₓ and higher AUC value than oral administration, thereby having faster and higher medicinal efficacy.

When substituting the bioavailability confirmed above, it can be confirmed that during intravenous administration of fexuprazan, it shows the same pK data at a dose 0.46 times that of oral administration. Therefore, it could be inferred that the therapeutic effect of oral administration of 40 mg of fexuprazan and intravenous administration of 18.4 mg, which is 0.46 times that amount, was at the same level.

### Experimental Example 6: Confirmation of PD value changes following multiple administration of fexuprazan injection

Subsequent to Experimental Example 4, in order to confirm the change in PD value during multiple administration of the compound of the present invention, the clinical trial was designed to be randomized, double-blind, placebo-controlled in healthy adults between the ages of 19 and 50.

A stabilizer (200 mg), a freeze-drying auxiliary agent (200 mg), and the previously prepared fexuprazan hydrochloride (40 mg based on fexuprazan weight) were dissolved in water for injection (4 mL), and the pH was adjusted to 4.0 by adding an appropriate amount of acid. The prepared solution was put into a vial and dried using a freeze-drying method to prepare a freeze-dried agent.

The freeze-dried composition prepared as above was used, but a total of 28 subject of 9, 8, and 8 subjects each were enrolled for three dose groups (20 mg, 40 mg, and 80 mg) based on fexuprazan weight, and each group was randomly assigned to test or control groups in the order that they passed the screening criteria at a ratio of 3:1.

**[Table 12]**

| | Dose(repeated intravenous administration) | Number of subject (test group: control group) |
|---|---|---|
| Dose group 1 | Fexuprazan (40 mg/vial) 20 mg IV q24h, 7 days | 9 (7 : 2) |
| Dose group 2 | Fexuprazan (40 mg/vial) 40 mg IV q12h, 7 days | 8 (6 : 2) |
| Dose group 3 | Fexuprazan (40 mg/vial) 80 mg IV q24h, 7 days | 8 (6 : 2) |

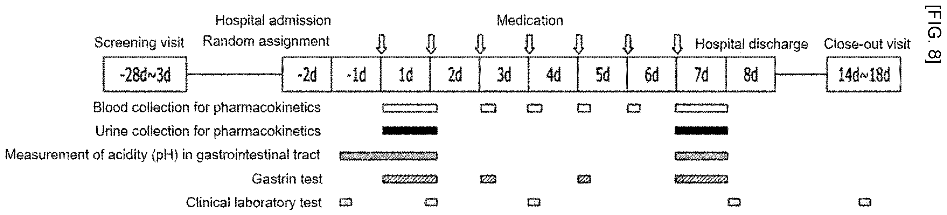

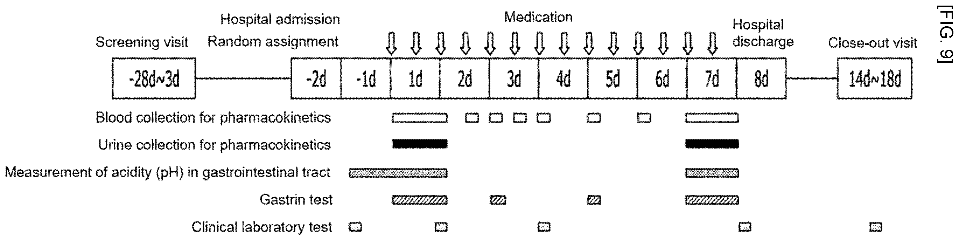

Dose groups 1 and 3 in Table 12 were administered once a day (every 24 hours) (FIG. 8), and dose group 2 was administered twice a day (every 12 hours) for 7 days (FIG. 9).

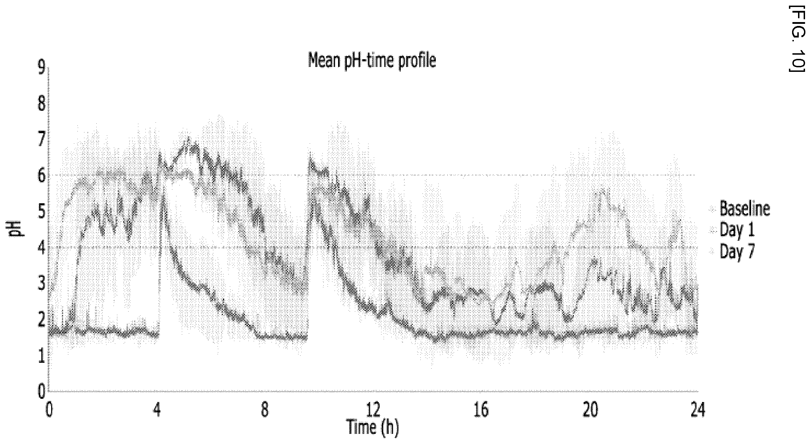

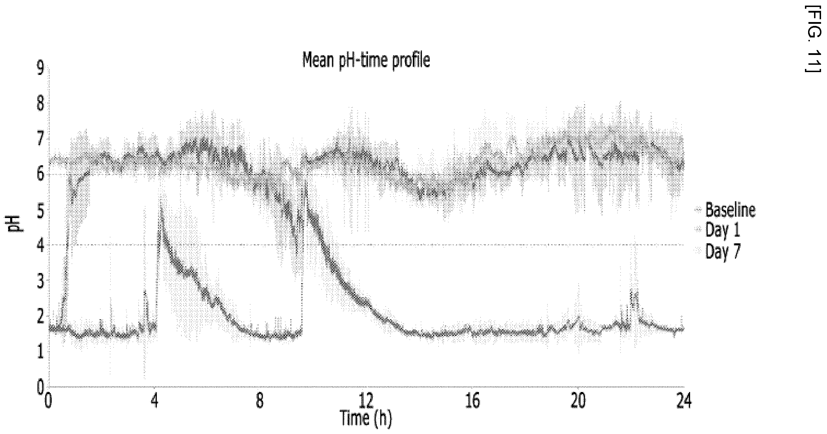

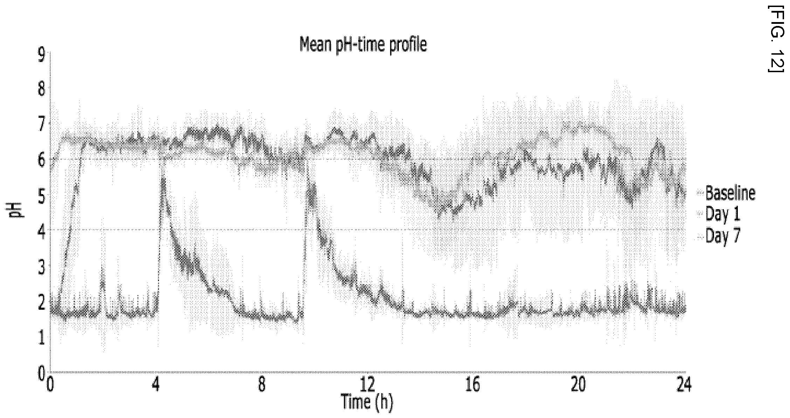

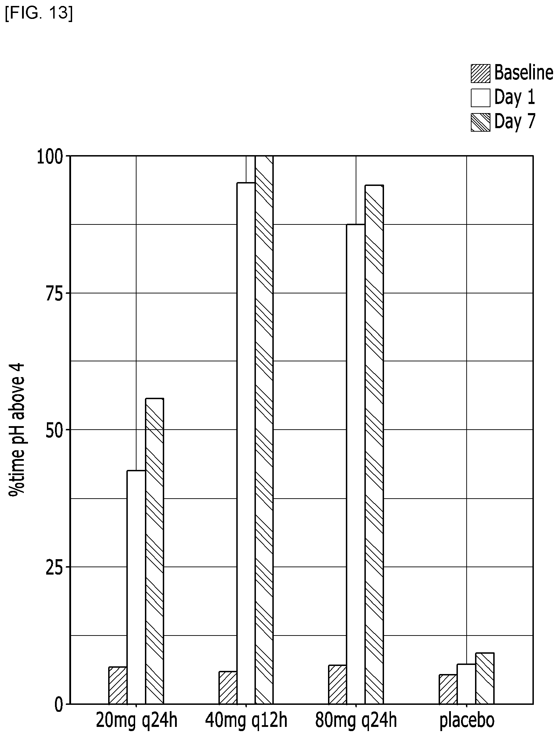

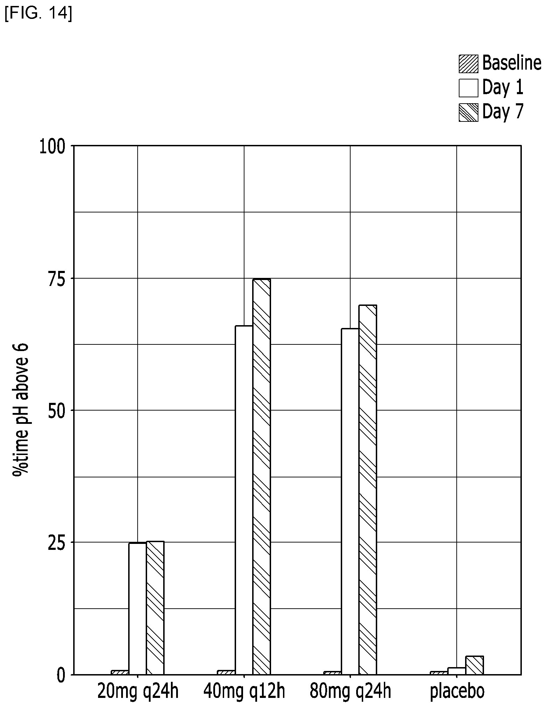

The acidity (pH) tests in the gastrointestinal tract were performed for 48 hours from the day before administration to 24 hours after the first administration. On the 7th day, it was administered for 24 hours after administration. The measured results are shown in Table 13 and FIGS. 10 to 14 below.

**[Table 13]**

| | 20mg q24h (n=6) | 40mg q12h (n=5) | 80mg q24h (n=6) | Placebo (n=6) |
|---|---|---|---|---|
| Fraction time pH≥6 over 24 hours post-dose (duration %)¹⁾ | 25.29 ± 15.41 | 74.77 ± 13.39 | 70.07 ± 18.09 | 3.33 ± 6.47 |
| Fraction time pH≥4 over 24 hours post-dose (duration %)¹⁾ | 55.68 ± 14.85 | 99.85 ± 0.31 | 94.78 ± 8.49 | 9.2 ± 5.66 |
| Mean pH | 4.28 ± 0.7 | 6.34 ± 0.23 | 6.08 ± 0.5 | 2.23 ± 0.32 |
| Median pH | 4.29 ± 1.32 | 6.33 ± 0.22 | 6.28 ± 0.27 | 1.77 ± 0.11 |
| 1) duration %, the fraction time during which the pH maintains above a certain value in 24 hours after administration (duration %) | | | | |

As can be seen from the above results, it is confirmed that when 20 mg was administered once a day, the time during which the pH in the gastrointestinal tract maintained above 4 on the 7th day was 55.68 (±14.85)%. It is confirmed that on the 7th day when 40mg was administered twice a day and 80mg was administered once a day, the time during which the pH in gastrointestinal tract maintained above 6 was 74.77 (±13.39)% and 70.07% (±18.09), respectively.

### Experimental Example 7: Confirmation of PD value changes according to fexuprazan injection maintenance therapy

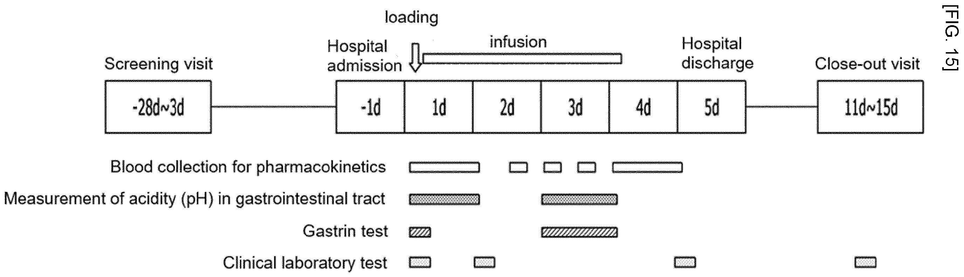

In order to confirm changes in PD values according to fexuprazan injection maintenance therapy, the test was conducted on eight healthy adults between the ages of 19 and 50. To the test subjects, a total of 100 ml of a solution obtained by mixing 40 mg of a freeze-dried preparation (40 mg/vial) prepared in the same manner as that used in Experimental Example 6, and physiological saline was administered intravenously for 30 minutes. Subsequently, on the first day, a total of 1L of a solution obtained by mixing 94 mg of freeze-dried formulation (40 mg/vial) and physiological saline was administered intravenously as a maintenance dose for 23.5 hours. On the second and third days, a total of 1L of a solution obtained by mixing 96 mg of freeze-dried formulation (40 mg/vial) and physiological saline was administered intravenously for 24 hours. That is, as a maintenance dose, a total of 3L of a solution of 286 mg of lyophilized formulation (40 mg/vial) mixed with physiological saline was administered intravenously for 71.5 hours at a rate of 4 mg/hr. The acidity in the gastrointestinal tract was tested on the first and third days, respectively, and the results are shown in Table 14 and FIG. 15 below.

**[Table 14]**

| | Day 1 | Day 3 |
|---|---|---|
| Fraction time pH≥6 over 24 hours post-dose (duration %)¹⁾ | 65.40 ± 18.67 | 76.33 ± 15.98 |
| Fraction time pH≥4 over 24 hours post-dose (duration %)¹⁾ | 97.87 ± 1.43 | 99.85 ± 0.40 |
| Mean pH | 6.17 ± 0.36 | 6.39 ± 0.35 |
| Median pH | 6.28 ± 0.32 | 6.44 ± 0.28 |
| 1) duration %, the fraction time during which the pH maintains above a certain value in 24 hours after administration (duration %) | | |

As can be seen from the above results, it is confirmed that when 40 mg was administered intravenously and maintained at 4 mg/h for 71.5 hours, the time during which the pH in the gastrointestinal tract maintained above 6 was 76.33 (±15.98)%, which makes it possible to predict the bleeding prevention effect of the corresponding therapy.

## Claims

1. An injection composition for preventing or treating gastrointestinal disease in which a compound represented by the following Chemical Formula 1 or a pharmaceutically acceptable salt thereof is administered at a dose of 0.4 to 0.6 times the dose at which the therapeutic effect for oral administration was confirmed:

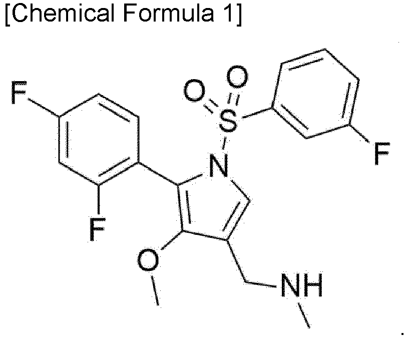

2. An injection composition for preventing or treating gastrointestinal disease in which a compound represented by the following Chemical Formula 1 or a pharmaceutically acceptable salt thereof is administered in an amount of 10 mg to 30 mg once a day:

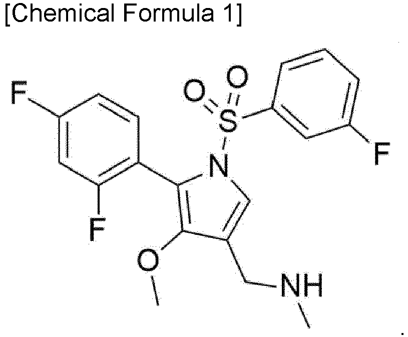

3. The injection composition according to claim 1 or 2, wherein:
the gastrointestinal disease is the disease associated with gastric acid secretion.

4. The injection composition according to claim 1 or 2, wherein:
the gastrointestinal disease is reflux esophagitis.

5. The injection composition according to claim 1 or 2, wherein:
the gastrointestinal disease is erosive esophagitis.

6. An injection composition for treating bleeding in which a compound represented by the following Chemical Formula 1 or a pharmaceutically acceptable salt thereof is administered in an amount of 70 mg to 90 mg once a day, or 30 mg to 50 mg twice a day:

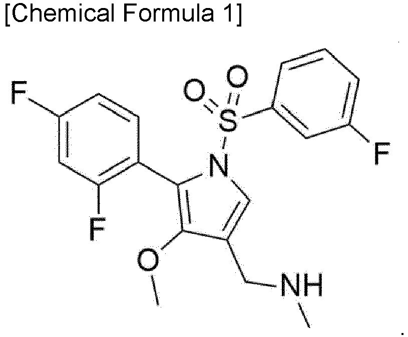

7. The injection composition according to claim 6, wherein:
the bleeding is caused by peptic ulcer.

8. The injection composition according to claim 6, wherein:
the bleeding is caused by a latrogenic ulcer after an endoscopic surgery.

9. An injection composition for preventing bleeding in which a compound represented by the following Chemical Formula 1 or a pharmaceutically acceptable salt thereof is administered in an amount of 30 mg to 50 mg, and then maintained and administered at 1 mg/h to 10 mg/h for 24 hours or more:
